# EUROPEAN PATENT SPECIFICATION

(11) **EP 3 107 568 B1**
(45) Date of publication and mention of the grant of the patent: **27.03.2024**
(21) Application number: 15752216.0
(22) Date of filing: 20.02.2015
(51) Int. Cl.: A61K 9/28, A61P 37/04, A61K 39/00

(54) **FORMULATIONS FOR SMALL INTESTINAL DELIVERY**
FORMULIERUNGEN FÜR KLEINE INTESTINALE VERABREICHUNG
FORMULATIONS POUR ADMINISTRATION DANS L'INTESTIN GRÊLE

(30) Priority: 20.02.2014 US 201461942386 P
(43) Date of publication of application: 28.12.2016
(60) Divisional application: 24158414.3
(73) Proprietor: Vaxart, Inc., South San Francisco, CA 94080 (US)
(72) Inventor: TUCKER, Sean, South San Francisco, California 94080 (US); TRAGER, George, South San Francisco, California 94080 (US)
(74) Representative: J A Kemp LLP
(86) International application number: PCT/US2015/016921
(87) International publication number: WO 2015/127278

(56) References cited:
- WO-A1-98/18453
- WO-A1-2011/026111
- WO-A1-2013/148258
- US-A- 4 152 413
- US-A1- 2001 043 949
- US-A1- 2003 133 950
- US-A1- 2010 221 329
- US-A1- 2013 273 154
- US-A1- 2013 337 055
- US-B1- 6 174 529
- Evonik Industries: "Eudragit", , 1 December 2015 (2015-12-01), XP055288214, Retrieved from the Internet: URL:http://eudragit.evonik.com/sites/lists /HN/Documents/evonik-brochure-eudragit-EN. pdf [retrieved on 2016-07-13]
- D F Evans ET AL: "Measurement of gastrointestinal pH profiles in normal ambulant human subjects.", Gut microbiota, vol. 29, no. 8, 1 August 1988 (1988-08-01) , pages 1035-1041, XP055651785, UK ISSN: 0017-5749, DOI: 10.1136/gut.29.8.1035

## Description

### CROSS-REFERENCES TO RELATED APPLICATIONS

The present application claims priority to US Provisional Application No. 61/942,386, filed February 20, 2014.

### BACKGROUND OF THE INVENTION

Vaccines are an important means for preventing and/or treating a number of diseases and disorders (e.g., viral infection, bacterial infection, and cancer). Vaccinization is typically carried out using injection, which reduces participation due to inconvenience of traveling to a vaccination site and aversion to injections. Furthermore, injection of vaccines requires use of sterile kit, such as syringes and needles, and a skilled practitioner to administer.

For the influenza vaccine, large-scale yearly campaigns are conducted to collect enough fertilized eggs to harvest and process sufficient virus to meet the needs of the market. Cell culture or plant derived hemagglutinin (HA) may reduce the burden of egg acquisition and processing, but these approaches still require expensive sterile fill and finish to produce individual syringe needles, that need to be disposed of as a biohazard. During a pandemic, schools can be closed and social distancing mandated, yet mass influenza immunization typically requires lining up subjects at health clinics for injections. Oral vaccines, for influenza or other pathogens, could be sent through the mail thus avoiding most human to human contact. Further, tableting is a rapid, sanitary process that does not require the expensive sterile fill and finishing process that injected vaccines require.

Vaccines that can be delivered in a non-parenteral manner, e.g., orally or mucosally, are described in US Patent No. 8,222,224.

WO 2013/148258 A1 discloses an oral vaccine formulation which delivers an antigen in the vicinity of the distal ileum of the gastrointestinal tract comprising an enteric coating which encapsulates the antigen, which is substantially insoluble at a pH of less than a range of between about 7.0 to about 7.6.

### BRIEF SUMMARY OF THE INVENTION

The references to methods of treatment in the summary and detailed description of the invention in this description are to be interpreted as references to the compounds, pharmaceutical compositions and medicaments of the present invention for use in a method for treatment of the human (or animal) body by therapy (or for diagnosis).

Provided herein are compositions and compositions for use for more effective vaccination of a subject (human or non-human) involving delivery of an immunogenic biological agent specifically to the ileum of the subject. The present disclosure thus provides more efficient and effective vaccines, and demonstrates their effectiveness in humans.

Provided herein are immunogenic compositions for eliciting an immune response in a subject comprising: (i) an adenoviral vector, encoding an immunogenic polypeptide encompassed by (ii) an agent that directs delivery of the adenoviral vector to the ileum of the human, wherein agent (ii) is an enteric coating that has a threshold pH 5.8, 5.9, 6, or 6.1.

In some embodiments, the subject is a human. In some embodiments, the subject is a non-human animal, *e.g.,* primate, mouse, rat, rabbit, horse, dog, cat, or poultry.

The immunogenic biological agent is an expression vector encoding an immunogenic polypeptide. The expression vector is an adenoviral vector. In some embodiments, the viral vector is attenuated or replication incompetent. In some embodiments, the expression vector comprises a promoter (*e.g.,* CMV, SV40 early or late, β-actin, etc.) operably linked to the sequence encoding the immunogenic polypeptide. In some embodiments, the expression vector further encodes double stranded (dsRNA). In some embodiments, the dsRNA encoding sequence is operably linked to a promoter, *e.g.,* either the same promoter (using an Internal Ribosomal Entry Site (IRES)) or a different promoter as the promoter operably linked to the immunogenic polypeptide encoding sequence.

In some embodiments, the immunogenic composition further comprises at least one adjuvant, *e.g.,* a TLR3 agonist. In some embodiments, the TLR3 agonist is dsRNA or a dsRNA mimetic.

In some embodiments, at least 50% of the immunogenic biological agent is delivered (released) in the ileum, *e.g.,* at least 60%, 70%, 75%, 80%, 90%, 95%, or more of the immunogenic biological agent present in the administered composition. In some embodiments, the agent that directs delivery (*e.g.,* the enteric coating or matrix) begins to dissolve before the immunogenic composition reaches the ileum, but retains at least 50% of the immunogenic biological agent until the immunogenic composition reaches the ileum. In some embodiments, the agent that directs delivery retains the immunogenic biological agent through the stomach, duodenum, and jejunum, but releases the immunogenic biological agent in the ileum.

The agent that directs delivery is an enteric coating. That is, the immunogenic biological agent is covered by an enteric coating. The enteric coating disintegrates at pH 5.8, 5.9, 6 or 6.1. In some embodiments, the enteric coating does not include cellulose acetate phthalate (CAP). The enteric coating is of a thickness that results in release of the immunogenic biological agent in the ileum. In some embodiments, the enteric coating is methacrylic acid copolymer-based with a coverage of 5.5-10 milligram per square centimeter. In some embodiments, the agent that directs delivery is a radio-controlled capsule.

In some embodiments, the enteric coating comprises poly(methacrylic acid-co-methyl methacrylate) 1:1. In some embodiments, the enteric coating comprises Eudragit^{®} L-100. In some embodiments, the enteric coating comprises Eudragit^{®} L-100, triethyl citrate, and talc, *e.g.,* 1, 2, 3, 4 or 1-4 parts Eudragit^{®} L-100, 1-2 parts triethyl citrate, and 1-2 parts talc.

In some embodiments, the immunogenic composition is in the form of a tablet or capsule, *e.g.,* in the form of a compressed tablet covered by enteric coating. In some embodiments, the immunogenic composition is encapsulated in a polymeric capsule comprising gelatin, hydroxypropylmethylcellulose, starch, or pullulan. In some embodiments, the immunogenic composition is in the form of microparticles less than 2mm in diameter, *e.g.,* each microparticle covered with enteric coating as described herein.

Further provided is a method of delivering an immunogenic composition to the ileum of a subject comprising orally administering the immunogenic composition as described above (*i.e.,* an immunogenic biological agent encompassed by an agent that directs delivery of the immunogenic biological agent to the ileum, optionally including an adjuvant) to the subject. In some embodiments, the subject is a human. In some embodiments, the subject is a non-human animal. In some embodiments, the method results in an immune response in the subject that is at least 10% higher, *e.g.,* at least 20%, 30%, 40%, 50%, 60%, 75%, 80%, 100% or more, than the immune response in a subject (either the same subject at a different time, or a different subject) receiving the same immunogenic composition not directed to the ileum. In some embodiments, the immune response in the subject is at least 1.5-fold higher (*e.g.,* 2-fold, 2.5-fold, 5-fold, or more) than the immune response in a subject (either the same subject at a different time, or a different subject) receiving the same immunogenic composition not directed to the ileum. In some embodiments, the immune response is an increase in antibodies specific for the immunogenic biological agent. In some embodiments, the immune response is a cellular immune response, *e.g.,* an increase in cytokines such as IFN-γ. In some embodiments, the immune response is immunization (*e.g.,* the subject is resistant to infection by the virus, bacteria, etc. from which the immunogenic biological agent was derived).

Further provided are methods of eliciting an increased immune response in a subject comprising orally administering the immunogenic composition as described above (*i.e.,* an immunogenic biological agent encompassed by an agent that directs delivery of the immunogenic biological agent to the ileum, optionally including an adjuvant) to the subject, *e.g.,* human subject. In some embodiments, the immune response is increased by at least 10%, *e.g.,* at least 20%, 30%, 40%, 50%, 60%, 75%, 80%, 100% or more, compared to the immune response in a subject (either the same subject at a different time, or a different subject) receiving the same immunogenic composition not directed to the ileum. In some embodiments, the immune response in the subject is increased at least 1.5-fold (*e.g.,* 2-fold, 2.5-fold, 5-fold, or more) compared the immune response in a subject (either the same subject at a different time, or a different subject) receiving the same immunogenic composition not directed to the ileum. In some embodiments, the immune response is an increase in antibodies specific for the immunogenic biological agent. In some embodiments, the immune response is a cellular immune response, *e.g.,* an increase in cytokines such as IFN-γ. In some embodiments, the immune response is immunization (*e.g.,* the subject is resistant to infection by the virus, bacteria, etc. from which the immunogenic biological agent was derived).

### BRIEF DESCRIPTION OF THE DRAWINGS

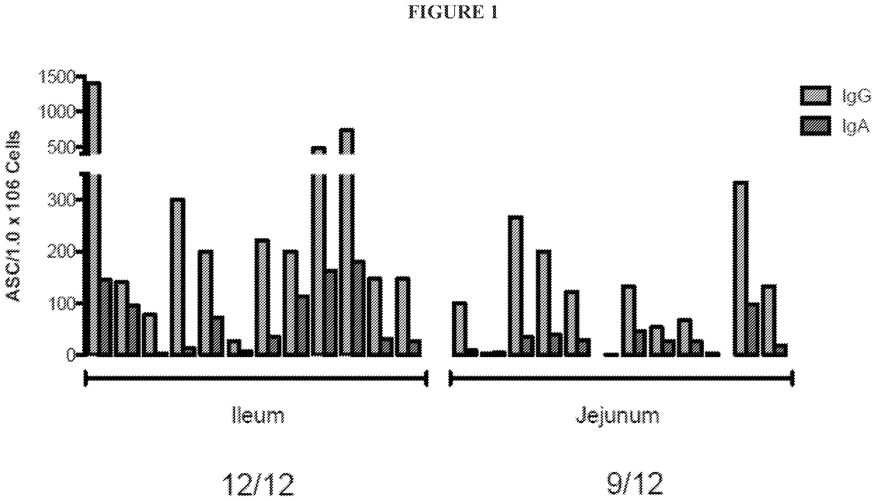

Figure 1. Antibody Secreting Cells (ASCs) specific for HA were measured in the peripheral blood 7 days after subjects were given a radio-controlled capsule containing rAd-HA-dsRNA. Subjects were randomized to have the vaccine released in either the ileum or jejunum. (N=12 per group). Results show that 12 of 12 subjects with vaccine delivered to the ileum were able to generate antibody secreting B cells that recognize HA, whereas only 9 of 12 subjects given the vaccine to the jejunum were able to generate antigen specific B cells. The average number of IgA and IgG ASCs was significantly higher for the ileum than the jejunum.

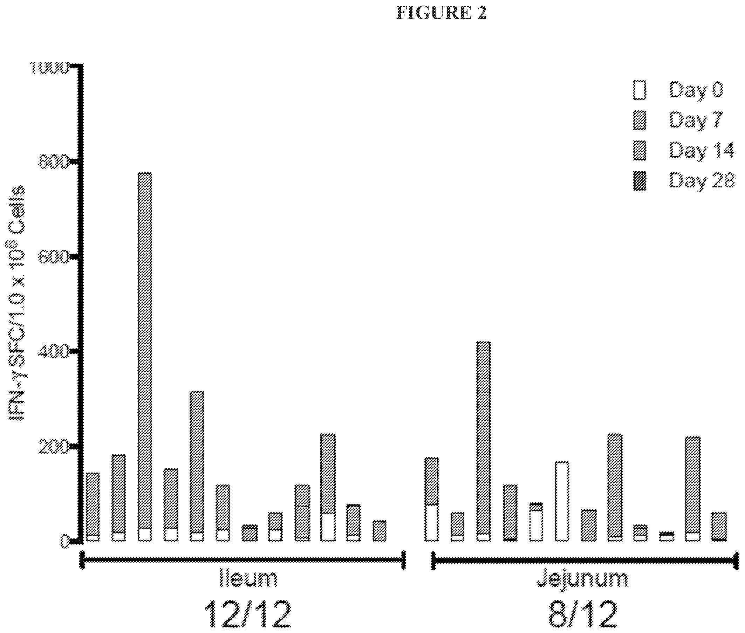

Figure 2. T cell response to rAd-HA-dsRNA was determined by detecting IFN-γ levels 7 days post-administration. All of the individuals in the ileum-delivery group showed higher levels of IFN-γ, compared to 75% of the jejunum-delivery group. The average IFN-γ level was also significantly higher in the ileum-delivery group.

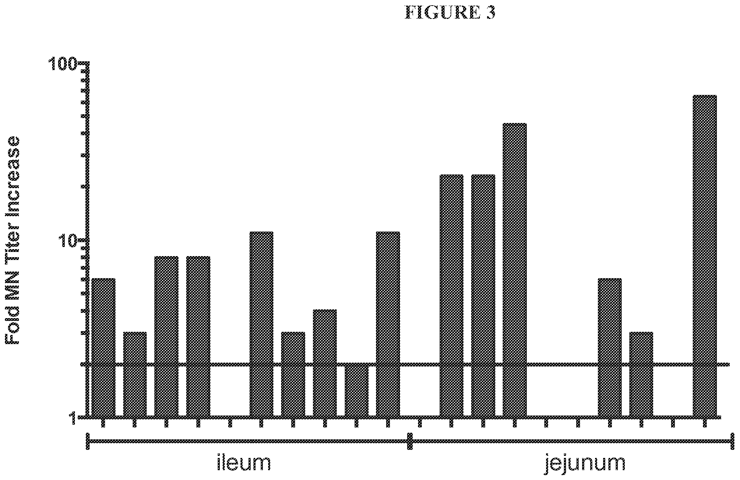

Figure 3. Microneutralizing antibody (MN) responses to influenza A/CA/07/2009 were measured at day 0 and day 28 after immunization. The fold increase in MN titers was plotted for individual subjects that had an initial MN titer less than or equal to 40. Results showed that ileum delivery resulted in a high proportion of subjects (9 of 10) with increased MN titers following immunization compared to jejunum delivery (6 of 10).

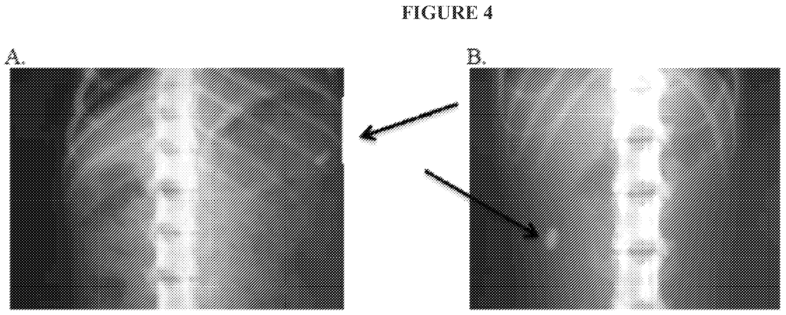

Figure 4. Tablets were made using microcrystalline cellulose and starch, with 10% barium sulfate as a radiopaque material. These tablets were enteric coated with Eudragit L100^{®} and given to female cynomolgus macaques by oral gastric tube. X-rays were taken over time post administration. A. Tablet in the stomach with the arrow pointing toward the tablet. B. One hour later, the tablet can be seen in the intestine, the white spot to the left of the spinal column with an arrow point toward it. It dissolved in the intestine within the next two hours, and cannot be seen.

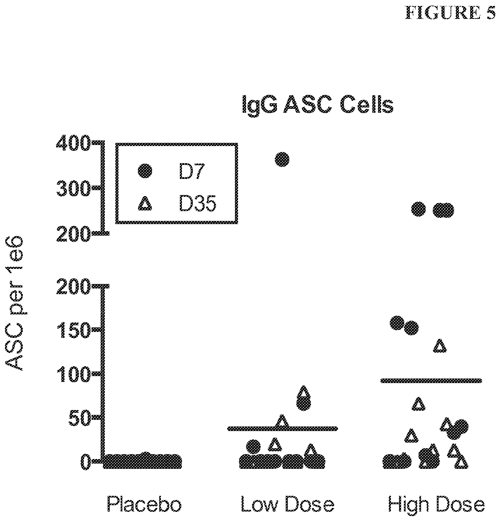

Figure 5. The numbers of ASCs are reported on days 7 and 35, 7 days after each immunization. Background ASCs at days 0 and 28 were miniscule, and not plotted. Average responses for day 7 are shown for each treated group with a horizontal line.

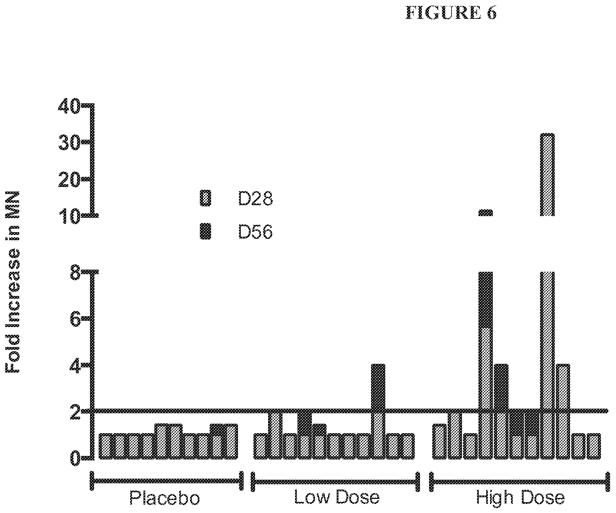

Figure 6. Fold increase in MN titers for individual subjects. The dark shaded columns indicate where the titers rose between days 28 and 56, whereas the light shaded columns shows the response after the initial immunization. A line was drawn at two fold increases in MN to show which subjects had a detectable neutralizing antibody response. No subject in the placebo group responded, whereas 3 subjects in the low dose and 7 subjects in the high dose group had a 2 fold or greater neutralizing antibody response to influenza after immunization. Placebo N=10, Low Dose and High Dose N=11.

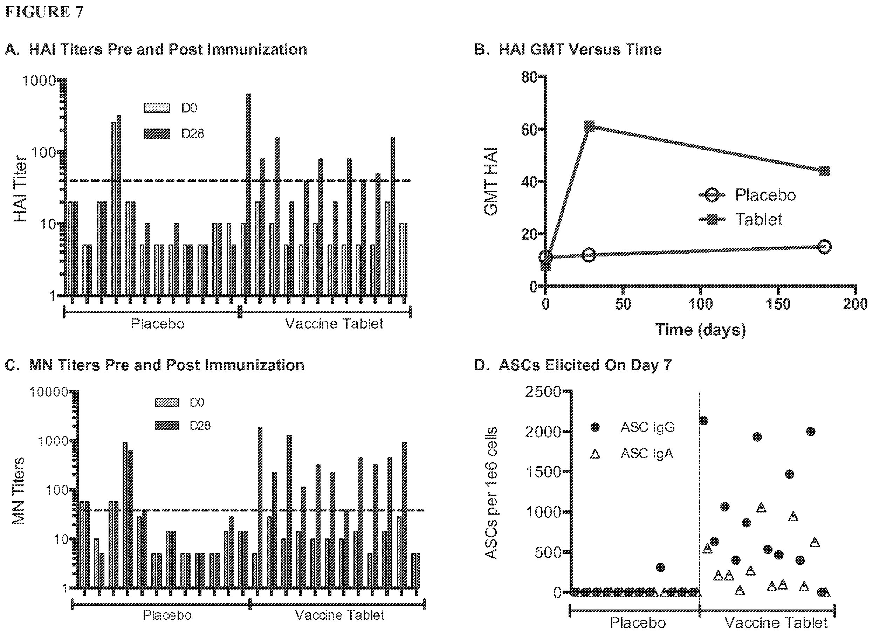

Figure 7. Antibody responses following a single oral immunization. A. HAI antibody titers pre and post immunization (days 0 and 28 respectively) are shown for individual subjects. B. HAI Geometric Mean Titers (GMT) vs Time. HAI titers were measured at 0, 1, and 6 months post immunization to evaluate the durability of the antibody response. C. MN titers, pre and post immunization are shown for individual subjects. D. ASC responses following immunization. The numbers of IgG and IgA ASCs are reported (per 10⁶ PMBCs) 7 days after immunization.

### DETAILED DESCRIPTION OF THE INVENTION

The inventors have discovered that delivery of an immunogenic biological agent to a particular part of the small intestine, *i.e.,* the ileum, results in a much greater therapeutic response than when the agent is not targeted, or is targeted to a different site. This allows for design of more effective vaccines, reduced costs for materials, and reduced side effects for the recipient.

### I. Definitions

The term "immunogenic" refers to the ability of an agent to give rise to an immune response in a host, either humoral or cell-mediated. Immunogenic agents are typically "foreign" to the host, *e.g.,* from a different species, or from a bacteria, virus, or fungus. A non-foreign agent can be immunogenic, e.g., in the case of an autoimmune response. Certain cancer cell-specific agents can be exploited as immunogenic agents, allowing the host's immune system to attack the cancer.

The term "biological agent" refers to a nucleic acid, polypeptide, glycoprotein, carbohydrate, lipid, or modified form thereof (*e.g.* methylated, glycosylated, detectably labeled). Biological agents are distinguished from small molecule drugs in that they can be created by biological processes (including recombinant techniques) instead of chemical synthesis. Biological agents can, however, be chemically modified or include non-natural nucleotides or amino acids. Biological agents can also be non-naturally occurring, *e.g.,* recombinant or chimeric entities.

As used herein, an "immunogenic biological agent" refers to an agent that acts directly as an antigen (*e.g.,* is recognized by a T cell receptor or antibody), or an agent that, once expressed in a cell, acts as an antigen. For example, an immunogenic biological agent can include an expression vector encoding an immunogenic polypeptide.

The term "antigen" refers to a polypeptide, glycoprotein, lipoprotein, lipid, carbohydrate, or other agent that is bound (*e.g.,* recognized as "foreign") by a T cell receptor and/or antibody. Antigens are commonly derived from bacterial, viral, or fungal sources. The term "derived from" indicates that the antigen is essentially as it exists in its natural antigenic context, or that it has been modified to be expressed under certain conditions, to include only the most immunogenic portion, or to remove other potentially harmful associated components, etc.

An "immunogenically effective dose or amount" of a composition as described herein is an amount that elicits or modulates an immune response specific for an antigen selected for vaccination. Immune responses include humoral immune responses and cell-mediated immune responses. An immunogenic composition can be used therapeutically or prophylactically to treat or prevent disease at any stage.

"Humoral immune responses" are mediated by cell free components of the blood, *e.g.,* plasma or serum; transfer of the serum or plasma from one individual to another transfers humoral immunity. Humoral immune responses are typically B cell-mediated, e.g., antibody production.

"Cell mediated immune responses" are mediated by antigen specific lymphocytes; transfer of the antigen specific lymphocytes from one individual to another transfers immunity. Cell-mediated immune responses are mediated at least in part by T cells, and can be detected, *e.g.,* by detecting T cell-specific cytokines or increase in T cell growth.

The "ileum" is the longest of the three segments that form the small intestine, along with the duodenum and jejunum. It is makes up the terminal portion, between the jejunum and cecum.

An enteric coating is a barrier applied to oral medications that prevents the therapeutic agent inside from being digested in the low pH environment of the stomach and duodenum (~pH 3).

An agent, such as an enteric coating, matrix, or capsule, is said to retain an encompassed or embedded therapeutic agent when at least 60%, *e.g.,* at least about 70%, 75%, 80%, 85%, 90%, 95%, or 100% of the original administered amount of therapeutic agent remains encompassed or embedded within the agent. The agent, *e.g.,* enteric coating or matrix, is typically designed to disintegrate under certain conditions and release the therapeutic agent. Disintegration can be gradual, *e.g.,* in the case of a thicker or more chemically complex coating. The enteric coating is said to "disintegrate" once the coating thickness is reduced at least 10%, *e.g.,* at least 25%, 50%, or 75% compared to the original administered thickness. Disintegration is not an absolute term, as it can occur over a different time course depending on conditions. For example, a coating that is designed to disintegrate in 5 minutes at pH 6.5 may disintegrate, albeit slowly, at pH 6 (e.g., in 1 hour). Disintegration does not necessarily indicate that the encompassed or embedded therapeutic agent is released. The therapeutic agent can, however, begin to be released before the enteric coating or matrix is entirely disintegrated.

The term "chimeric" or "recombinant" as used herein with reference, *e.g.,* to a nucleic acid, protein, or vector, indicates that the nucleic acid, protein or vector, has been modified by the introduction of a heterologous nucleic acid or protein or the alteration of a native nucleic acid or protein. Thus, for example, chimeric and recombinant vectors include nucleic acid sequences that are not found within the native (non-chimeric or non-recombinant) form of the vector. A chimeric viral expression vector refers to a viral expression vector comprising a nucleic acid sequence encoding a heterologous (*e.g.,* immunogenic) polypeptide.

An "expression vector" is a nucleic acid construct, generated recombinantly or synthetically, with a series of specified nucleic acid elements that permit transcription of a particular nucleic acid in a host cell. The expression vector can be part of a plasmid, virus, or nucleic acid fragment. Typically, the expression vector includes a nucleic acid to be transcribed operably linked to a promoter. Viral expression vectors are typically rendered replication incompetent or attenuated. A virally-derived vector can include the components of the expression vector required for expression of a desired sequence, but omit those involved in, *e.g.,* replication or other pathogenic effects.

The terms "promoter" and "expression control sequence" are used herein to refer to a nucleic acid control sequence that directs transcription of a nucleic acid. Promoter sequences are typically near the start site of transcription, such as a TATA element in the case of a polymerase II type promoter. A promoter can also include distal enhancer or repressor elements, which can be located as much as several thousand base pairs from the start site of transcription. Promoters include constitutive and inducible promoters. A "constitutive" promoter is a promoter that is active under most environmental and developmental conditions. An "inducible" promoter is a promoter that is active under environmental or developmental regulation. The term "operably linked" refers to a functional linkage between a nucleic acid expression control sequence (such as a promoter, or array of transcription factor binding sites) and a second nucleic acid sequence, wherein the expression control sequence directs transcription of the nucleic acid corresponding to the second sequence.

The term "heterologous" when used with reference to portions of a nucleic acid indicates that the nucleic acid comprises two or more subsequences that are not found in the same relationship to each other in nature. For instance, the nucleic acid is typically recombinantly produced, having two or more sequences from unrelated genes arranged to make a new functional nucleic acid, *e.g.,* a promoter from one source and a coding region from another source. Similarly, heterologous portions of a protein indicates that the protein comprises two or more subsequences that are not found in the same relationship to each other in nature (e.g., a fusion protein). A heterologous nucleic acid or protein is one that is not found in a particular environment in nature, *e.g.,* a heterologous mouse protein in a human cell.

The terms "nucleic acid" and "polynucleotide" are used interchangeably herein to refer to polymers of deoxyribonucleotides or ribonucleotides in either single- or double-stranded form. The terms encompass genes, cDNA, RNA, and oligonucleotides (short polynucleotides). The terms encompass nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphonates, 2-O-methyl ribonucleotides, peptide-nucleic acids (PNAs). The term "nucleotide" typically refers to a nucleic acid monomer.

Unless otherwise indicated, a particular nucleic acid sequence also encompasses conservatively modified variants thereof (*e.g.,* degenerate codon substitutions) and complementary sequences, as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); Rossolini et al., Mol. Cell. Probes 8:91-98 (1994)).

A "therapeutic dose" or "therapeutically effective amount" or "effective amount" of a composition as described herein is an amount that prevents, alleviates, abates, or reduces the severity of symptoms of diseases and disorders associated with the source of the antigen selected for vaccination (e.g., a virus, bacteria, a parasite, or a cancer).

The term "antibody" refers to a polypeptide encoded by an immunoglobulin gene or fragments thereof that specifically bind and recognizes an antigen. Immunoglobulin sequences include the kappa, lambda, alpha, gamma, delta, epsilon, and mu constant region sequences, as well as myriad immunoglobulin variable region sequences. Light chains are classified as either kappa or lambda. Heavy chains are classified as gamma, mu, alpha, delta, or epsilon, which in turn define the immunoglobulin classes, IgG, IgM, IgA, IgD and IgE, respectively.

T cells refer to a particular class of lymphocytes that express a specific receptor (T cell receptor) encoded by a family of genes. The recognized T cell receptor genes include alpha, beta, delta, and gamma loci, and the T cell receptors typically (but not universally) recognize a combination of MHC plus a short peptide. T cells are typically broadly classified as T helper cells (CD4+) and cytotoxic T cells (CD8+). Antibodies are naturally produced by B cells, *e.g.,* Antibody Secreting Cells (ASCs). Mature B cells can be naive, plasma B cells (activated and antibody-producing), memory, B-1, marginal-zone B cells, follicular B cells, and regulatory B cells.

An adaptive immune response refers to T cell and/or B cell and/or antibody recognition of antigen.

Antigen presenting cells (APCs) are cells that are able to present immunogenic peptides or fragments thereof to T cells to activate or enhance an immune response. APCs include dendritic cells, macrophages, B cells, monocytes and other cells that may be engineered to be efficient APCs. Such cells may, but need not, be genetically modified to increase the capacity for presenting the antigen, to improve activation and/or maintenance of the T cell response, to have anti-tumor effects per se and/or to be immunologically compatible with the receiver (*i.e.,* matched HLA haplotype). APCs may be isolated from any of a variety of biological fluids and organs including bone marrow, peripheral blood, tumor and peritumoral tissues, and may be autologous, allogeneic, syngeneic or xenogeneic cells. APCs typically utilize a receptor from the major histocompatability (MHC) locus to present short polypeptides to T cells.

An adjuvant is a non-specific immune response enhancer. Suitable adjuvants include, for example, cholera toxin, monophosphoryl lipid A (MPL), Freund's Complete Adjuvant, Freund's Incomplete Adjuvant, Quil A, and Al(OH). Adjuvants can also be those substances that cause APC activation and enhanced presentation of T cells through secondary signaling molecules like Toll-like receptors, e.g., double-stranded RNA (dsRNA), dsRNA mimetics, bacterial flagella, LPS, CpG DNA, and bacterial lipopeptide (Reviewed recently in [Abreu et al., J Immunol, 174(8), 4453-4460 (2005)]).

The terms "polypeptide," "peptide" and "protein" are used interchangeably herein to refer to a polymer of amino acids. The terms apply to amino acid polymers in which one or more amino acid residue is an artificial chemical mimetic of a corresponding naturally occurring amino acid, as well as to naturally occurring amino acid polymers and non-naturally occurring amino acid polymer.

The term "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, *e.g.,* hydroxyproline, γ-carboxyglutamate, and O-phosphoserine. Amino acid analogs refers to compounds that have the same basic chemical structure as a naturally occurring amino acid, *i.e.,* a carbon that is bound to a hydrogen, a carboxyl group, an amino group, and an R group, *e.g.,* homoserine, norleucine, methionine sulfoxide, methionine methyl sulfonium. Such analogs have modified R groups (*e.g.,* norleucine) or modified peptide backbones, but retain the same basic chemical structure as a naturally occurring amino acid. Amino acid mimetics refers to chemical compounds that have a structure that is different from the general chemical structure of an amino acid, but that functions in a manner similar to a naturally occurring amino acid.

Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

"Conservatively modified variants" applies to both amino acid and nucleic acid sequences. With respect to particular nucleic acid sequences, conservatively modified variants refers to those nucleic acids which encode identical or essentially identical amino acid sequences, or where the nucleic acid does not encode an amino acid sequence, to essentially identical sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. Every nucleic acid sequence herein which encodes a polypeptide also describes every possible silent variation of the nucleic acid. One of skill will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid which encodes a polypeptide is implicit in each described sequence.

As to amino acid sequences, one of skill will recognize that individual substitutions, deletions or additions to a nucleic acid, peptide, polypeptide, or protein sequence which alters, adds or deletes a single amino acid or a small percentage of amino acids in the encoded sequence is a "conservatively modified variant" where the alteration results in the substitution of an amino acid with a chemically similar amino acid. Conservative substitution tables providing functionally similar amino acids are well known in the art. Such conservatively modified variants are in addition to and do not exclude polymorphic variants, interspecies homologs, and alleles of the invention.

The following eight groups each contain amino acids that are conservative substitutions for one another: 1) Alanine (A), Glycine (G); 2) Aspartic acid (D), Glutamic acid (E); 3) Asparagine (N), Glutamine (Q); 4) Arginine I, Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M) (see, e.g., Creighton, Proteins (1984)).

The phrase "selectively (or specifically) hybridizes to" refers to the binding, duplexing, or hybridizing of complementary (or largely complementary) nucleotides in a complex mixture (e.g., total cellular or library DNA or RNA).

Polynucleotides may comprise a native sequence (*i.e*., an endogenous sequence that encodes an individual polypeptide or dsRNA or a portion thereof) or may comprise a variant of such a sequence. Polynucleotide variants may contain one or more substitutions, additions, deletions and/or insertions such that at least one biological activity of the encoded polypeptide (*e.g.,* immunogenicity) is not diminished, relative to a polypeptide comprising native antigens. Polynucleotide variants may contain one or more substitutions, additions, deletions and/or insertions such that the adjuvant activity of an encoded dsRNA is not diminished, relative to a dsRNA that does not contain the substitutions, additions, deletions and/or insertions. Variants preferably exhibit at least about 50%, 55%, 60%, 65%, 70%, 75%, 80%, 85%, 90%, 91%, 92%, 93%, 94%, 95%, 96%, 97%, 98%, or 99% sequence identity to a polynucleotide sequence that encodes a native polypeptide or a portion thereof or a dsRNA.

The terms "identical" or percent "identity," in the context of two or more polynucleotide or polypeptide sequences, refer to two or more sequences or subsequences that are the same or have a specified percentage of amino acid residues or nucleotides that are the same (*i.e.,* 50%, 55%, 60%, 65%, 70%, 75%, 80%, 85%, 90%, 91%, 92%, 93%, 94%, 95%, 96%, 97%, 98%, 99%, or more identity over a specified region), when compared and aligned for maximum correspondence over a comparison window, or designated region as measured using one of the following sequence comparison algorithms or by manual alignment and visual inspection. Such sequences are then said to be "substantially identical." This definition also refers to the compliment of a test polynucleotide sequence. Optionally, the identity exists over a region that is at least about 10 to about 100, about 20 to about 75, about 30 to about 50 amino acids or nucleotides in length.

A "control" sample or value refers to a sample that serves as a reference, usually a known reference, for comparison to a test sample. For example, a test sample can be taken from a test condition, *e.g.,* in the presence of a test compound or treatment, and compared to samples from known conditions, *e.g.,* in the absence of the test compound (negative control), or in the presence of a known compound (positive control). In the context of the present disclosure, an example of a negative control would be a biological sample from a known healthy (non-infected) individual, and an example of a positive control would be a biological sample from a known infected patient. A control can also represent an average value or a range gathered from a number of tests or results. One of skill in the art will recognize that controls can be designed for assessment of any number of parameters. For example, a control can be devised to compare therapeutic benefit based on pharmacological data (*e.g.,* half-life) or therapeutic measures (*e.g.,* comparison of benefit and/or side effects). Controls can be designed for *in vitro* applications. One of skill in the art will understand which controls are valuable in a given situation and be able to analyze data based on comparisons to control values. Controls are also valuable for determining the significance of data. For example, if values for a given parameter are widely variant in controls, variation in test samples will not be considered as significant.

The term "diagnosis" refers to a relative probability that a subject has a disorder such as an infection or cancer. Similarly, the term "prognosis" refers to a relative probability that a certain future outcome may occur in the subject. The terms are not intended to be absolute, as will be appreciated by any one of skill in the field of medical diagnostics.

The terms "therapy," "treatment," and "amelioration" refer to any reduction in the severity of symptoms. In the context of infection, treatment can refer to a reduction of infectious agent, reduced symptoms, etc. In the case of treating cancer, treatment can refer to, e.g., reducing tumor size, number of cancer cells, growth rate, metastatic activity, reducing cell death of non-cancer cells, etc. The terms "treat" and "prevent" are not intended to be absolute terms. Treatment and prevention can refer to any comparative reduction or apparent absence of infectious agent, delay in onset, amelioration of symptoms, improvement in patient survival, increase in survival time or rate, etc. Treatment and prevention can be complete (undetectable levels of infectious agent or neoplastic cells) or partial, such that fewer infectious agent or neoplastic cells are found in a patient than would have occurred without the presently described immunogenic biological agents. The effect of treatment can be compared to an individual or pool of individuals not receiving the treatment, or to the same patient prior to treatment or at a different time during treatment. In some aspects, the severity of infection or disease is reduced by at least 10%, as compared, *e.g.,* to the individual before administration or to a control individual not undergoing treatment. In some aspects the severity of infection or disease is reduced by at least 25%, 50%, 75%, 80%, or 90%, or in some cases, no longer detectable using standard diagnostic techniques.

"Subject," "patient," "individual" and like terms are used interchangeably and refer to, except where indicated, mammals such as humans and non-human primates, as well as rabbits, rats, mice, goats, pigs, and other mammalian species. The term does not necessarily indicate that the subject has been diagnosed with a particular disease, but typically refers to an individual under medical supervision. A patient can be an individual that is seeking treatment, monitoring, adjustment or modification of an existing therapeutic regimen, etc.

### II. Immunogenic biological agents

An immunogenic biological agent is any biological agent that causes an immune response in the host, *e.g.,* human host. The immunogenic biological agent can thus be a polypeptide (*e.g.,* glycoprotein, phosphoprotein, or other modified form), carbohydrate, lipid, polynucleotide (*e.g.,* chromatin, methylated polynucleotide, or other modified form). In some embodiments, the immunogenic biological agent directly causes an immune response, *e.g.,* is itself a target immunogen (antigen). In some embodiments, the immunogenic biological agent is a polynucleotide encoding the target immunogen. For example, when a polynucleotide encoding a target antigen is expressed in an antigen presenting cell (APC), an immune response is mounted against the expressed antigen. The immunogenic biological agent can be administered alone, in combination with a second, third, and/or fourth immunogenic biological agent (*e.g.,* in the case of a multi-target preventative vaccine), and/or in combination with an adjuvant to increase the immune response.

### A. Expression vectors

Expression vectors for use as described herein can include virally-derived vectors, *e.g.,* recombinant adeno-associated virus (AAV) vectors, retroviral vectors, adenoviral vectors, modified vaccinia Ankara (MVA) vectors, and lentiviral (*e.g.,* HSV-1-derived) vectors (*see, e.g.,* Brouard et al. (2009) British J. Pharm. 157:153). Virally-derived vectors for therapeutic use are typically rendered replication incompetent or attenuated. For example, in the case of an adenoviral vector, the adenoviral genome can be modified to remove the E1 and E3 genes. For production, the replication deficient vector can be administered to a cell that expresses the E1 gene such that recombinant adenovirus (rAd) is produced by the cell. This rAd can be harvested and used for a single round of infection to deliver the transgenic composition to another cell within a mammal in order to elicit immune responses to an encoded polypeptide antigen.

Examples of suitable viral vectors include adenovirus 5, including, for example, Ad5 with deletions of the E1/E3 regions and Ad5 with a deletion of the E4 region. Other suitable adenoviral vectors include strains 2, orally tested strains 4 and 7, enteric adenoviruses 40 and 41, and other strains (*e.g.* Ad34, Ad26, or Ad35) that are sufficient for delivering an antigen and eliciting an adaptive immune response to the transgene antigen [Lubeck et al., Proc Natl Acad Sci USA, 86(17), 6763-6767 (1989); Shen et al., J Virol, 75(9), 4297-4307 (2001); Bailey et al., Virology, 202(2), 695-706 (1994)]. The viral vector does not need to have been isolated from humans, but can come from a non-human such as chimpanzee adenovirus 3 (ChAd3) (*see, e.g.,* Colloca et al. (2012) Sci. Transl. Med. 4:115; Stanley et al. (2014) Nat. Med. doi:10.1038/nm.3702). In some embodiments, the adenoviral vector is a live, replication incompetent adenoviral vector (such as E1 and E3 deleted rAd5), live and attenuated adenoviral vector (such as the E1B55K deletion viruses), or a live adenoviral vector with wild-type replication.

Transcriptional and translational control sequences in expression vectors to be used as described herein can be provided by viral sources. For example, commonly used promoters and enhancers are derived, *e.g.,* from beta actin, adenovirus, simian virus (SV40), and human cytomegalovirus (CMV). For example, vectors allowing expression of proteins under the direction of the CMV promoter, SV40 early promoter, SV40 later promoter, metallothionein promoter, murine mammary tumor virus promoter, Rous sarcoma virus promoter, transducer promoter, or other promoters shown effective for expression in mammalian cells are suitable. Additional viral and non-viral promoter, control and/or signal sequences may be used, provided such control sequences are compatible with the host cells to be transfected.

### B. Immunogens

Immunogens for use as described herein can be derived from antigens, such as, for example, viral antigens, bacterial antigens, cancer antigens, fungal antigens, or parasite antigens (*see, e.g.,* US Patent No. 8,222,224 for a list of antigens that can be used as described herein).

Particular examples of antigens that can be used as described herein are those derived from the influenza virus (e.g., HA, NA, M1, NP), human immunodeficiency virus (HIV, *e.g.,* gag, pol, env, etc.), human papilloma virus (HPV, *e.g.,* capsid proteins such as L1), Venezuelan Equine Encephalomyelitis (VEE) virus, Epstein Barr virus, herpes simplex virus (HSV), human herpes virus, rhinoviruses, cocksackieviruses, enteroviruses, hepatitis A, B, C, E, and G (HAV, HBV, HCV, HEV, HGV *e.g.,* surface antigen), mumps virus, rubella virus, measles virus, poliovirus, smallpox virus, rabies virus, and Varicella-zoster virus.

Suitable viral antigens also include viral nonstructural proteins, *e.g.,* proteins encoded by viral nucleic acid that do not encode for structural polypeptides, in contrast to those that make capsid or the protein surrounding a virus. Non-structural proteins include those proteins that promote viral nucleic acid replication, viral gene expression, or post-translationsal processing, such as, for example, Nonstructural proteins 1, 2, 3, and 4 (NS1, NS2, NS3, and NS4, respectively) from Venezuelan Equine encephalitis (VEE), Eastern Equine Encephalitis (EEE), or Semliki Forest.

Bacterial antigens can be derived from, for example, Staphylococcus aureus, Staphylococcus epidermis, Helicobacter pylori, Streptococcus bovis, Streptococcus pyogenes, Streptococcus pneumoniae, Listeria monocytogenes, Mycobacterium tuberculosis, Mycobacterium leprae, Corynebacterium diphtheriae, Borrelia burgdorferi, Bacillus anthracis, Bacillus cereus, Clostridium botulinum, Clostridium difficile, Salmonella typhi, Vibrio chloerae, Haemophilus influenzae, Bordetella pertussis, Yersinia pestis, Neisseria gonorrhoeae, Treponema pallidum, Mycoplasm sp., Legionella pneumophila, Rickettsia typhi, Chlamydia trachomatis, and Shigella dysenteriae, Vibrio cholera (e.g., Cholera toxin subunit B, cholera toxin-coregulated pilus (TCP)); Helicobacter pylorii (*e.g.,* VacA, CagA, NAP, Hsp, catalase, urease); E. coli (*e.g.,* heat-labile enterotoxin, fimbrial antigens).

Parasite antigens can be derived from, for example, Giardia lamblia, Leishmania sp., Trypanosoma sp., Trichomonas sp., Plasmodium sp. (*e.g.,* P. falciparum surface protein antigens such as pfs25, pfs28, pfs45, pfs84, pfs 48/45, pfs 230, Pvs25, and Pvs28); Schistosoma sp.; Mycobacterium tuberculosis (e.g., Ag85, MPT64, ESAT-6, CFP10, R8307, MTB-32 MTB-39, CSP, LSA-1, LSA-3, EXP1, SSP-2, SALSA, STARP, GLURP, MSP-1, MSP-2, MSP-3, MSP-4, MSP-5, MSP-8, MSP-9, AMA-1, Type 1 integral membrane protein, RESA, EBA-175, and DBA).

Fungal antigens can be derived from, for example, Tinea pedis, Tinea corporus, Tinea cruris, Tinea unguium, Cladosporium carionii, Coccidioides immitis, Candida sp., Aspergillus fumigatus, and Pneumocystis carinii.

Cancer antigens include, for example, antigens expressed or over-expressed in colon cancer, stomach cancer, pancreatic cancer, lung cancer, ovarian cancer, prostate cancer, breast cancer, skin cancer (*e.g.,* melanoma), leukemia, or lymphoma. Exemplary cancer antigens include, for example, HPV L1, HPV L2, HPV E1, HPV E2, placental alkaline phosphatase, AFP, BRCA1, Her2/neu, CA 15-3, CA 19-9, CA-125, CEA, Hcg, urokinase-type plasminogen activator (Upa), plasminogen activator inhibitor, CD53, CD30, CD25, C5, CD11a, CD33, CD20, ErbB2, CTLA-4. *See* Sliwkowski & Mellman (2013) Science 341:6151 for additional cancer targets.

### C. Adjuvants

In some embodiments, the compositions further comprise at least one adjuvant. Suitable adjuvants include, for example, the lipids and non-lipid compounds, cholera toxin (CT), CT subunit B, CT derivative CTK63, E. coli heat labile enterotoxin (LT), LT derivative LTK63, Al(OH)₃, and polyionic organic acids as described in *e.g.,* WO2004/020592, Anderson and Crowle, Infect. Immun. 31(1):413-418 (1981), Roterman et al., J. Physiol. Pharmacol., 44(3):213-32 (1993), Arora and Crowle, J. Reticuloendothel. 24(3):271-86 (1978), and Crowle and May, Infect. Immun. 38(3):932-7 (1982)). Suitable polyionic organic acids include for example, 6,6'-[3,3'-demithyl[1,1'-biphenyl]-4,4'-diyl]bis(azo)bis[4-amino-5-hydrox- y-1,3-naphthalene-disulfonic acid] (Evans Blue) and 3,3'-[1,1' biphenyl]-4,4'-diylbis(azo)bis[4-amino-1-naphthalenesulfonic acid] (Congo Red). It will be appreciated by those of skill in the art that the polyionic organic acids may be used for any nucleic acid-based vaccination method in conjunction with any type of administration.

TLR-3 agonists (*e.g.,* dsRNA, and mimetics thereof such as polyI:C, poly A:U, and polyI:polyC) can also be used. TLR-3 agonists include, for example, short hairpin RNA, virally derived RNA, short segments of RNA that can form double-strands or short hairpin RNA, and short interfering RNA (siRNA). In some embodiments, the TLR-3 agonist is virally derived dsRNA, *e.g.,* dsRNA derived from a Sindbis virus or dsRNA viral intermediates (Alexopoulou et al. (2001) Nature 413:732). In some embodiments, the TLR-3 agonist is a short hairpin RNA. Short hairpin RNA sequences typically comprise two complementary sequences joined by a linker sequence. The particular linker sequence is not a critical aspect of the invention. Any appropriate linker sequence can be used so long as it does not interfere with the binding of the two complementary sequences to form a dsRNA. TLR-3 agonists can result in pro-inflammatory cytokine release (*e.g.* IL-6, IL-8, TNF-alpha, IFN-alpha, IFN-beta) when contacted with a responder cell (*e.g.,* a dendritic cell, a peripheral blood mononuclear cell, or a macrophage) in vitro or in-vivo.

Other suitable adjuvants include topical immunomodulators such as, members of the imidazoquinoline family such as, for example, imiquimod and resiquimod (*see, e.g.,* Hengge et al., Lancet Infect. Dis. 1(3):189-98 (2001).

Additional suitable adjuvants are commercially available as, for example, additional alum-based adjuvants (*e.g.,* Alhydrogel, Rehydragel, aluminum phosphate, Algammulin); oil based adjuvants (Freund's Incomplete Adjuvant and Complete Adjuvant (Difco Laboratories, Detroit, Mich.), Specol, RIBI, TiterMax, Montanide ISA50 or Seppic MONTANIDE ISA 720); nonionic block copolymer-based adjuvants, cytokines (*e.g.,* GM-CSF or Flat3-ligand); Merck Adjuvant 65 (Merck and Company, Inc., Rahway, N.J.); AS-2 (SmithKline Beecham, Philadelphia, Pa.); salts of calcium, iron or zinc; an insoluble suspension of acylated tyrosine; acylated sugars; cationically or anionically derivatized polysaccharides; polyphosphazenes; biodegradable microspheres; monophosphoryl lipid A and Quil A. Cytokines, such as GM-CSF or interleukin-2, -7, or -12, are also suitable adjuvants. Hemocyanins (*e.g.,* keyhole limpet hemocyanin) and hemoerythrins can also be used as adjuvants. Polysaccharide adjuvants such as, for example, chitin, chitosan, and deacetylated chitin are also suitable as adjuvants. Other suitable adjuvants include muramyl dipeptide (MDP, N acetylmuramyl L alanyl D isoglutamine) bacterial peptidoglycans and their derivatives (*e.g.,* threonyl-MDP, and MTPPE). BCG and BCG cell wall skeleton (CWS) can be used as adjuvants, with or without trehalose dimycolate. Trehalose dimycolate can be used itself (*see, e.g.,* US Patent No. 4,579,945). Detoxified endotoxins are also useful as adjuvants alone or in combination with other adjuvants (*see, e.g.,* US Patent Nos. 4,866,034; 4,435,386; 4,505,899; 4,436,727; 4,436,728; 4,505,900; and 4,520,019). The saponins QS21, QS17, QS7 are also useful as adjuvants (*see, e.g.,* US Patent No. 5,057,540; EP 0362 279; WO 96/33739; and WO 96/11711). Other suitable adjuvants include Montanide ISA 720 (Seppic, France), SAF (Chiron, Calif., United States), ISCOMS (CSL), MF-59 (Chiron), the SBAS series of adjuvants (e.g., SBAS-2, SBAS-4 or SBAS-6 or variants thereof, available from SmithKline Beecham, Rixensart, Belgium), Detox (Corixa, Hamilton, Mont.), and RC-529 (Corixa, Hamilton, Mont.).

Superantigens are also contemplated for use as adjuvants in the present invention. Superantigens include Staphylococcus exoproteins, such as the alpha, beta, gamma, and delta enterotoxins from S. aureus and S. epidermidis, and the alpha, beta, gamma, and delta E. coli exotoxins. Common Staphylococcus enterotoxins are known as staphylococcal enterotoxin A (SEA) and staphylococcal enterotoxin B (SEB), with enterotoxins through E (SEE) being described (Rott et al., 1992). Streptococcus pyogenes B (SEB), Clostridium perfringens enterotoxin (Bowness et al., 1992), cytoplasmic membrane-associated protein (CAP) from S. pyogenes (Sato et al., 1994) and toxic shock syndrome toxin 1 (TSST 1) from S. aureus (Schwab et al., 1993) can also be used.

For the pharmaceutical compositions provided herein, the adjuvant(s) can be designed to induce, *e.g.,* an immune response predominantly of the Th1 or Th2 type. High levels of Th1-type cytokines (*e.g.,* IFN-gamma, TNF-alpha, IL-2 and IL-12) tend to favor the induction of cell mediated immune responses to an administered antigen. In contrast, high levels of Th2-type cytokines (*e.g.,* IL-4, IL-5, IL-6 and IL-10) tend to favor the induction of humoral immune responses. Following oral delivery of a composition comprising an immunogenic polypeptide as provided herein, an immune response that includes Th1- and Th2-type responses will typically be elicited.

### III. Targeted delivery systems

The presently described compositions and methods for ileal delivery can rely on appropriate coatings, matrices, and devices such as those described below.

### A. Enteric coatings, matrices, and devices

Enteric coatings are used to shield substances from the low pH environment of the stomach and delay release of the enclosed substance until it reaches a desired target later in the digestive tract. Enteric coatings are known, and commercially available. Examples include pH-sensitive polymers, bio-degradable polymers, hydrogels, time-release systems, and osmotic delivery systems (*see, e.g.,* Chourasia & Jain (2003) J. Pharm. Pharmaceutical Sci. 6:33).

The pH of the gastrointestinal tract (GIT) progresses from very acidic in the stomach (pH ~2), to more neutral in the ileum (pH ~ 5.8-7.0). pH sensitive coatings can be used that dissolve in the ileum or just before the ileum. Examples include Eudragit^{®} L and S polymers (threshold pH's ranging from 5.5-7.0); polyvinyl acetate phthalate (pH 5.0), hydroxypropyl methylcellulose phthalate 50 and 55 (pH 5.2 and 5.4, respectively), and cellulose acetate phthalate (pH 5.0). Thakral et al. (2013) Expert Opin. Drug Deliv. 10:131 review Euragit^{®} formulations for ileal delivery, in particular, combinations of L and S that ensure delivery at pH≤7.0. Crotts et al. (2001) Eur. J Pharm. Biol. 51:71 describe Eudragit^{®} formulations with appropriate disintegration properties. Vijay et al. (2010) J. Mater. Sci. Mater. Med. 21:2583 review acrylic acid (AA)-methyl methacrylate (MMA) based copolymers for ileal delivery at pH 6.8.

For ileal delivery, the polymer coating typically dissolves at about pH 6.8 and allows complete release within about 40 min (*see, e.g.,* Huyghebaert et al. (2005) Int. J. Pharm. 298:26). To accomplish this, a therapeutic substance can be covered in layers of different coatings, *e.g.,* so that the outermost layer protects the substance through low pH conditions and is dissolved when the tablet leaves the stomach, and at least one inner layer that dissolves as the tablet passes into increasing pH. Examples of layered coatings for delivery to the distal ileum are described, *e.g.,* in WO2013148258.

Biodegradable polymers (*e.g.,* pectin, azo polymers) typically rely on the enzymatic activity of micro flora living in the GIT. The ileum harbors larger numbers of bacteria than earlier stages, including lactobacilli and enterobacteria.

Osmotic-controlled Release Oral delivery Systems (OROS^{®}; Alza) is an example of an osmotic system that degrades over time in aqueous conditions. Such materials can be manipulated with other coatings, or in varying thicknesses, to deliver specifically to the ileum (*see, e.g.,* Conley et al. (2006) Curr. Med. Res. Opin. 22:1879).

Combination polymers for delivery to the ileum are reported in WO2000062820. Examples include Eudragit^{®} L100-55 (25 mg/ capsule) with triethyl citrate (2.4 mg/ capsule), and Povidone K-25 (20 mg/ tablet) followed by Eudragit^{®} FS30D (30 mg/ tablet). pH sensitive polymers can be applied to effect delivery to the ileum, as described above and, *e.g.,* methacrylic acid copolymers (*e.g.,* poly(methacylic acid-co-methyl methacrylate) 1:1), cellulose acetate phthalate, hydroxypropyl methylcellulose phthalate, hydroxypropyl methylcellulose acetate succinate, polyvinyl acetate phthalate, cellulose acetate trimellitate, carboxymethyl ethyl- cellulose, shellac or other suitable polymer(s). The coating layer can also be composed of film-forming polymers being sensitive to other luminal components than pH, such as bacterial degradation or a component that has such a sensitivity when it is mixed with another film-forming polymer. Examples of such components providing delayed release to the ileum are polymers comprising azo bond(s), polysaccharides such as pectin and its salts, galactomannans, amylose and chondroitin, disulphide polymers and glycosides.

Components with varying pH, water, and enzymatic sensitivities can be used in combination to target a therapeutic composition to the ileum. The thickness of the coating can also be used to control release. The components can also be used to form a matrix, in which the therapeutic composition is embedded. See generally, Frontiers in Drug Design & Discovery (Bentham Science Pub. 2009) vol. 4.

### B. Frequency or radio-controlled capsules

As an alternative to dissolving coatings and matrices, site-specific delivery can be via capsules that release upon an externally generated signal. Early models released for a high-frequency (HF) signal, as disclosed in Digenis *et al.* (1998) *Pharm. Sci. Tech. Today* 1:160. The original HF capsule concept has since been updated and the result marketed as InteliSite^{®}. The updated capsule is a radio-frequency activated, non-disintegrating delivery system. Radiolabeling of the capsule permits the determination of the capsule location within a specific region of the GI tract via gamma scintigraphy. When the capsule reaches the desired location in the GI tract, external activation opens a series of windows to the capsule drug reservoir.

In some embodiments, the immunogenic biological agent can be enclosed in a radio-controlled capsule, so that the capsule is tracked and signaled once it reaches the ileum. In some embodiments, the capsule is signaled at a given time after administration that corresponds to when the capsule is expected to arrive in the ileum, with or without detecting.

### C. Formulations

Pharmaceutical compositions can be used for prophylactic and therapeutic purposes as described herein. As explained above, pharmaceutical compositions can be prepared to protect against stomach degradation such that the administered immunogenic biological agent reach the desired location. Methods for microencapsulation of DNA and drugs for oral delivery are described, *e.g.,* in US2004043952.

An immunogenic pharmaceutical composition can contain pharmaceutically acceptable salts of the immunogenic biological agent (*e.g.,* immunogenic polypeptide, or polynucleotide encoding an immunogenic polypeptide). Such salts may be prepared from pharmaceutically acceptable non-toxic bases, including organic bases (*e.g.,* salts of primary, secondary and tertiary amines and basic amino acids) and inorganic bases (*e.g.,* sodium, potassium, lithium, ammonium, calcium and magnesium salts). Some particular examples of salts include phosphate buffered saline and saline (*e.g.,* for ingestion, nasal delivery, or injection).

A delayed release coating or an additional coating of the formulation can contain other film-forming polymers being non-sensitive to luminal conditions for technical reasons or chronographic control of the drug release. Materials to be used for such purpose includes, but are not limited to; sugar, polyethylene glycol, polyvinylpyrrolidone, polyvinyl alcohol, polyvinyl acetate, hydroxypropyl cellulose, methylcellulose, ethylcellulose, hydroxypropyl methylcellulose, carboxymethylcellulose sodium and others, used alone or in mixtures.

Additives such as dispersants, colorants, pigments, additional polymers, *e.g.,* poly(ethylacrylat, methylmethacrylat), anti-tacking and anti-foaming agents can be included into a coating layer. Other compounds may be added to increase film thickness and to decrease diffusion of acidic gastric juices into the core material. The coating layers can also contain pharmaceutically acceptable plasticizers to obtain desired mechanical properties. Such plasticizers are for instance, but not restricted to, triacetin, citric acid esters, phthalic acid esters, dibutyl sebacate, cetyl alcohol, polyethylene glycols, glycerol monoesters, polysorbates or other plasticizers and mixtures thereof. The amount of plasticizer can be optimised for each formula, and in relation to the selected polymer(s), selected plasticizer(s) and the applied amount of said polymer(s).

Other suitable pharmaceutical ingredients known in the art can be employed in the pharmaceutical compositions of this invention. Suitable carriers include, for example, water, saline, alcohol, a fat, a wax, a buffer, a solid carrier, such as mannitol, lactose, starch, magnesium stearate, sodium saccharine, talcum, cellulose, glucose, sucrose, and magnesium carbonate, or biodegradable microspheres (*e.g.,* polylactate polyglycolate). Suitable biodegradable microspheres are disclosed, for example, in US Patent Nos. 4,897,268; 5,075,109; 5,928,647; 5,811,128; 5,820,883. The immunogenic polypeptide and/or carrier expression vector can be encapsulated within the biodegradable microsphere or associated with the surface of the microsphere.

Such compositions may also comprise non-immunogenic buffers (*e.g.,* neutral buffered saline or phosphate buffered saline), carbohydrates (*e.g.,* glucose, mannose, sucrose or dextrans), mannitol, proteins, polypeptides or amino acids such as glycine, antioxidants, bacteriostats, chelating agents such as EDTA or glutathione, adjuvants (*e.g.,* aluminum hydroxide), suspending agents, thickening agents and/or preservatives. Alternatively, compositions of the present invention may be formulated as a lyophilate. Compounds may also be encapsulated within liposomes using well known technology.

### IV. Immune responses and vaccines

The pharmaceutical compositions for ileum delivery as described herein are designed to elicit an immune response from an individual that is specific for an immunogenic biological agent included in the pharmaceutical composition. The pharmaceutical composition can be used prophylactically or therapeutically as a vaccine to avoid or reduce a viral infection, bacterial infection, parasitic infection, fungal infection, or cancer. The pharmaceutical compositions can be used to treat at any stage, *e.g.,* at the pre-cancer, cancer, or metastatic stages, or to prevent disease or infection.

For example, the compositions described herein may be used to prevent or treat infection, such as influenza, hepatitis, or HIV, or for prevention or treatment of cancer. Within such methods, pharmaceutical compositions are typically administered to an individual that may or may not be afflicted with the disease, disorder, or infection. In some embodiments, a disease, disorder, or infection is diagnosed prior to administration, *e.g.,* using criteria generally accepted in the art. For example, viral infection may be diagnosed by the measurement of viral titer in a sample from the patient, bacterial infection may be diagnosed by detecting the bacteria in a sample from the patient, and cancer may be diagnosed by detecting the presence of a malignant tumor. Pharmaceutical compositions can be administered either prior to or following surgical removal of primary tumors and/or treatment such as administration of radiotherapy or conventional chemotherapeutic drugs.

Immunotherapy is typically active immunotherapy, in which treatment relies on the in vivo stimulation of the endogenous host immune system to react against, *e.g.,* tumors or bacterially or virally infected cells, with the administration of immune response-modifying agents (*e.g.,* immunogenic biological agents).

Frequency of administration of the prophylactic or therapeutic compositions described herein, as well as dosage, will vary from individual to individual, and can be readily established using standard techniques. Typically, between 1 and 52 doses can be administered over a 52 week period. In some embodiments, 3 doses are administered, at intervals of 1 month, or 2-3 doses are administered every 2-3 months. In some embodiments, a combination of more than one antigen can be administered simultaneously or sequentially, *e.g.,* an annual influenza vaccine that contains individual components directed at each subtype of influenza or multiple clades within a subtype. In some embodiments, the intervals are more like once a year, *e.g.,* an annual flu vaccine based on the particular current strain. Booster vaccinations can be given periodically thereafter. Alternate protocols may be appropriate for individual patients and particular diseases and disorders.

A suitable dose is an amount of an immunogenic biological agent that, when administered as described above, is capable of promoting, *e.g.,* an anti-tumor, an anti-viral, or an antibacterial, immune response, and is at least 15-50% above the basal (untreated) level, or at least 5-50% (*e.g.,* 5%, 10%, 20%, 30%, 50%, 1.5-fold, 2-fold, or higher) above the level from non-ileum targeted treatment. Such response can be monitored by measuring the anti-tumor antibodies in a patient or by vaccine-dependent generation of cytolytic T cells capable of killing, *e.g.,* the patient's tumor cells, the patient's virally infected cells, or the patient's bacterially infected cells *in vitro.* Such vaccines can also generate an immune response that leads to an improved clinical outcome (*e.g.,* complete or partial or longer disease-free survival, reduced viral titers) in vaccinated patients as compared to non-vaccinated patients, or patients receiving non-ileum targeted treatment.

In general, an appropriate dosage and treatment regimen provides the active compound(s) in an amount sufficient to provide therapeutic and/or prophylactic benefit. Such a response can be monitored by establishing an improved clinical outcome (*e.g.,* reduced or negative viral titer, more frequent remissions, complete or partial, or longer disease-free survival) in treated patients as compared to patients treated with non-ileum targeted treatment, or non-treated patients. Such immune responses can generally be evaluated using standard proliferation, cytotoxicity or cytokine assays described above, which can be performed using samples obtained from a patient before and after treatment.

For example, detection of immunocomplexes formed between an immunogenic polypeptide and antibodies in body fluid that are specific for the immunogenic polypeptide can be used to monitor the effectiveness of therapy, *e.g.,* for a disease or disorder in which the immunogenic polypeptide is associated. Samples of body fluid taken from an individual prior to and subsequent to initiation of therapy (*e.g.,* ileum-targeted therapy) may be analyzed for the immunocomplexes using known methods. Briefly, the number of immunocomplexes detected in both samples is compared. A significant change in the number of immunocomplexes in the second sample (post-targeted therapy) relative to the first sample (pre-targeted therapy) reflects successful therapy.

### V. Examples

Pharmaceutical methods for delivering small molecules to the intestine are known, but the ability to deliver a large biological to the intestine for proper immune recognition is poorly understood. Mice are not able to swallow pills, so it is difficult to perform studies with tablets in animal models. Further, the location of the best place to deliver the vaccine vector in order to elicit a response to transgene antigen has not been characterized in humans. In sheep, the jejunum was shown to be the most effective target for eliciting an immune response to an adenovirally-encoded transgene antigen (Mutwari *et al.* (1999) *Immunology* 97:455). Here we show the result of several human or non-human primate studies with improved human oral dosage forms for delivery of biological agents.

### Example 1

In order to determine which region of the small intestine is most active for inducing an immune response to antigen, tests were performed in humans. Radio-controlled capsules were given to healthy normal volunteers, with the vaccine either released early in the small intestine (jejunum) or later in the small intestine (ileum). The use of the radio-controlled capsules for delivery of small molecule drugs has been described, but not for vaccine delivery (Digenis et al. (1991) Crit. Rev. Ther. Drug Carrier Syst. 7:309).

The vaccine was composed of recombinant adenovirus expressing the influenza antigen HA from A/CA/04/2009 (rAd-HA-dsRNA) (*see, e.g.,* US2012/0244185). A total of 10¹¹ infectious units (IU) were given to each subject on day 0. The numbers of circulating pre-plasma B cells in peripheral blood were measured by Antibody Secreting Cell (ASC) assay on days 0 and 7 after the administration of the vaccine. Results only measure the numbers of ASCs that recognize the antigen HA.

Results show that ASCs could be measured 7 days after immunization in each of the treated groups (Figure 1). Average responses were higher in the ileum dosed group than the jejunum dosed group. Background ASCs on day 0 were negligible. For the ileum, an average of 340 +/- 111 (standard error) IgG and 74 +/- 18 IgA ASCs were observed on day 7. For the jejunum, the average and standard error responses were 118 +/- 30 IgG and 28 +/- 8 IgA ASCs. The ileum group was significantly different than placebo (P=0.03 on day 7 for IgA ASC, and trended higher for IgG ASC p=0.07). Contrary to the results in sheep, the results in humans indicate that ileum delivery is more potent at eliciting an IgG or an IgA antibody response than jejunum delivery.

T cell responses were also determined by detecting interferon-γ release (IFN-γ) using the ELISPOT^{®} assay. Figure 2 shows that 12/12 of the ileum-dosed group had increased levels of IFN-γ, compared to 8/12 of the jejunum-dosed group 7 days post-administration. In addition, IFN-γ levels were significantly higher in the ileum-dosed group than in the jejunum-dosed group.

Microneutralizing (MN) antibody titers to influenza A/CA/07/2009 were measured. Increased MN antibody levels are indicative of a neutralizing antibody response. After excluding subjects that had an initial neutralizing antibody response greater than 40 (Faix *et al.* (2012) *PloS One 7:e34581*)*,* the fold increases in MN titers were plotted for individual subjects. The number of subjects with a positive increase was 9 out of 10 for the ileum delivered vaccine versus 6 out of 10 for jejunum delivered vaccine (Figure 3). The geometric mean titers (GMT) were similar between the two groups, with ileum GMT rising from 22 to 92 versus the jejunum GMT rising from 18 to 90. The results indicate that ileum release is more reliable at inducing neutralizing antibody responses to influenza, possibly leading to a greater percentage of subjects protected against influenza.

### Example 2

Tablets were hand made using microcrystalline cellulose (PH-101, FMC) and starch (Starch 1500, Colorcon) incorporating 10% barium sulfate as a radiopaque material containing fumed silica as a flow aid and magnesium stearate as a tablet lubricant. The tablets of 7.14 mm diameter and 150 mg weight were coated with Eudragit^{®} L-100 in a pan coater using 10% coating solids weight gain as a guide to whether the enteric coating was added; coating solids contained 4 parts Eudragit^{®} polymer to one part triethyl citrate and 1 part talc. As an initial test of enteric coating performance, four cynomolgus macaques were given tablets using an oral gastric tube. The oral gastric tube is solid and rigid, but hollow down the middle for instilling liquids. It has a flexible silicone tube on the leading end of the rigid tube that can hold a small tablet in place. The tube and pill apparatus were threaded down the esophagus of restrained monkeys until the leading end passed through the cardiac sphincter and into the stomach. A flush of orange juice was used to dislodge the pill into the stomach. X-rays were taken at set time points, and examined for location and dissolution of the tablet. Table 1 summarizes the results.

| **Table 1: L-100 coating performance** | | | | |
|---|---|---|---|---|
| **Animal** | **Time and Pill Location** | | | |
| | 1 hr | 2 hr | 3 hr | 4hr |
| 1 | stomach | stomach | stomach | intestine |
| 2 | stomach | intestine | intestine | dissolved |
| 3 | intestine | intestine | dissolved | dissolved |
| 4 | stomach | stomach | intestine | dissolved |

Figure 4 shows that the tablets were completely intact in the low pH environment of the stomach; there was no evidence of premature dissolution of the tablets. While large for a monkey, the tablets were able to pass through the stomach intact into the intestine. In the intestine, they dissolved at a reasonable rate and were completely dissolved in 3 out of 4 monkeys. In the 4^{th} monkey, the pill left the stomach sometime after 3 hours and the pill had not dissolved at the time of the last x-ray. Overall, the tablets performed in an acceptable manner and the Eudragit^{®} L-100 coating was selected for future human studies.

### Example 3

A phase 1, sequentially enrolled clinical study, with a randomized and placebo-controlled cohort to evaluate safety, and immunogenicity of a recombinant Ad serotype 5 (rAd5) based oral vaccine against H1 seasonal influenza was completed. The rAd5 vector (rAd-HA-dsRNA with HA from A/CA/04/2009) was described in Example 1. The study had an active phase of approximately 3 months, and was conducted in accordance with applicable Good Clinical Practice guidelines, the United States Code of Federal Regulations, and the International Conference on Harmonization guidelines. Informed consent was obtained from all subjects after discussion of the risks. IRB approval was given before dosing of subjects.

Good manufacturing practice (GMP)-grade rAd-HA-dsRNA was produced in Wave^{®} bags (GE Healthcare, Waukesha, WI) at Lonza Biologicals (Houston, TX). Purification was performed by ion exchange chromatography, followed by buffer exchange. Purified vector was mixed with excipients, lyophilized, and then tableted at Lonza using microcrystalline cellulose and starch as tableting bulk. Tablets were enteric coated with Eudragit^{®} L 100 (Evonik Industries, Darmstadt, Germany) using a Vector HiCoater^{®} LDCS-5 coater (Vector Freund, Cedar Rapids, IA). The final product was released in one lot, and titered by standard IU assay. Placebo was prepared as similarly sized and shaped tablets containing 150 mg of microcrystalline cellulose, without enteric coating. The study compared 10⁹ IU, 10¹⁰ IU, and placebo treated subjects for the ability to elicit an immune response to transgene. Subjects were given tablets on days 0 and 28.

The numbers of circulating pre-plasma B cells in peripheral blood were measured by ASC assays on days 0 and 7 after the initial dose, and at days 28 and 35 after the second dose (the second dose was delivered at day 28). Results show that ASC counts could be measured 7 days after each immunization in the treated groups, but not the placebo group (Figure 5). Average responses were higher on day 7, and higher in the high dose group than the low dose group. Background ASCs on days 0 and 28 were negligible, and negligible for the placebo group at all time points. For the high dose group, an average of 105 +/- 33 and 27 +/- 12 ASCs were found for days 7 and 35 respectively. For the low dose group, average ASCs were 41 +/- 32 and 14 +/- 8 for days 7 and 35 respectively. The placebo group had an average of 0.3 +/- 0.3 and 0, for days 7 and 35 respectively. The high dose group was significantly higher than placebo (P=0.01 and 0.05 for days 7 and 35 respectively.)

Neutralizing antibody responses to influenza were measured by MN assay. Results show a dose dependent increase in the MN titers in the treated groups versus the placebo control (Figure 6). The frequency of MN responders with at least a 2-fold increase in the high dose group was significantly different than the placebo group (P=0.003 by Fisher's exact test), whereas the low dose trended higher, but was not significantly higher than placebo (P=0.2). After removing subjects that had MN titers greater than 40, the geometric mean titers (GMT) were calculated in the remaining subjects (Table 2). Day 56 Geometric Fold Titer Response (GMFR) was also calculated (Table 2). These results show that neutralizing antibody titers to influenza are being generated by oral immunization, with a greater than 3 fold increase in the GMT after immunization in the high dose group. These results show that L 100 coated tablets can be used for vaccine delivery to the intestine.

| **Table 2: GMT changes in MN titres for subjects with MN≤ 40** | | | | | |
|---|---|---|---|---|---|
| **Group** | **N** | **GMT D0** | **GMT D28** | **GMT D56** | **GMFR** |
| Placebo | 8 | 14.1 | 14.1 | 14.1 | 1 |
| Low Dose | 10 | 12.3 | 14.1 | 16.2 | 1.3 |
| High Dose | 7 | 15.6 | 36.2 | 53.8 | 3.4 |

### Example 4

We tested parameters for enteric coatings *in vitro* to determine dissolution times with varying pH and coating percentage. The data provide guidelines for ileal delivery following gastric exposure at low pH (as in the stomach) and subsequent transit through an increasing pH gradient (as is found in the duodenum and jejunum) prior to reaching the ileum.

Tablet disintegration was tested with 150 mg tablets prepared as described above, and coated with 8, 10, or 12% total solids weight gain, utilizing Eudragit^{®} L100, Eudragit^{®} L100-55, or 1:1 (w/w) mixture of L100 and L100-55 polymers, applied as an organic solvent suspension. In duplicate, tablets prepared with each coating polymer, and at each level of coating application, were pre-exposed to USP simulated gastric fluid (SGF, pH 1.6, no pepsin) for 120 minutes in a VanKel Bio-Dis III reciprocating cylinder dissolution test apparatus at 37 °C at a reciprocation rate of 10 dips per minute (DPM). The tablets were then transferred to USP simulated intestinal fluid (SIF, pH 6.8, no pancreatin). Tablets were observed for disintegration and the time to complete disintegration of both tablets was recorded to the nearest 5 minutes. The data indicate that disintegration time is influenced by both polymer composition and thickness and provide guidance with regard to proper selection of coating composition to influence the behavior of the coatings after tablets exit the stomach.

| Coating Polymer | Time to disintegrate at indicated coating level (minutes) | | |
|---|---|---|---|
| | 8% | 10% | 12% |
| L100 | 20 | 30 | 45 |
| L100/L100-55 | 15 | 20 | 30 |
| L100-55 | 10 | 20 | 25 |

The effect of pH on disintegration time was tested with 150 mg tablets, coated to 10% total solids weight gain with either Eudragit^{®} L100 or Eudragit^{®} L100-55. A series of buffers were prepared by adjusting the pH of USP SIF (no pancreatin) to values encompassing the USP specification of 6.8. Tablets were pre-exposed to USP SGF (no pepsin) for 120 minutes 37 °C and 10 DPM, then transferred to the pH-modified USP SIF solutions. The tablets were observed for disintegration and the time to complete disintegration was recorded to the nearest 5 minutes. The data indicate that the rate of disintegration is influenced by the environmental pH and differs between the two polymers. Again, the results can be used for proper selection of a coating composition to accomplish drug retention through the stomach and upper small intestine.

| pH of SIF | Time to disintegrate for polymer at pH (minutes) | |
|---|---|---|
| | Eudragit L100 | Eudragit L100-55 |
| 5.4 | 250 | 145 |
| 6 | 110 | 60 |
| 6.4 | 55 | 45 |
| 7 | 30 | 20 |

### Example 5

We carried out a Phase 1, sequentially enrolled study, with a randomized and placebo-controlled cohort to evaluate safety, and immunogenicity of a recombinant Ad serotype 5 (rAd5) based oral vaccine against H1 seasonal influenza. Tablets containing the vaccine were coated as described herein to dissolve in the ileum. The data show that an oral tablet vaccine would be competitive with existing vaccines in terms of eliciting neutralizing antibody responses to influenza.

Hemagglutination Inhibition (HAI) responses were measured on days 0 and 28 (Figure 7A). No placebo treated subject seroconverted, but one placebo subject slipped through screening and had a high day 0 value. None of the vaccine subjects had a starting HAI titer > 20. After immunization, nine subjects in the vaccine group reached seroprotective levels (HAI ≥40) (Figure 7A). The Geometric Mean Titer (GMT) for the group was 61·1 (95% CI: 30 - 124), a 7.7-fold geometric mean fold rise (GMFR) over the initial GMT of 7·9 (95% CI: 6 - 11). Of the eleven 4-fold risers (92%), nine seroconverted (SC) with the other 2 subjects showing a 4-fold increase in HAI titer from 5 to 20. The vaccine group had a statistically significant increase in the number of 4-fold responders versus placebo (11 versus 0, with P<0·0000 by Fisher's Exact Test). The placebo subjects had a GMT of 11.9 (95% CI: 6 - 25) on day 28 versus a GMT of 11.0 on day 0 (95% CI: 5 - 23).

Durability of the antibody response was measured by examining HAI responses 180 days after immunization. In the vaccine-immunized group, 75% (9 of 12) of the subjects were seroprotected on day 28 and 75% (9 of 12) were still seroprotected on day 180. The HAI GMT were plotted (Figure 7B), and the decrease in the GMT was found to be 28% between 28 and 180 days post immunization.

Neutralizing antibody responses to influenza were measured by MN assay. Significant increases in the MN titers in the treated group versus the placebo control were observed (Figure 7C). The frequency of 4-fold MN responders in the vaccine treated group was significantly different than the placebo group, with 11 subjects responding in the vaccine treated group versus 0 in the placebo group (P<0·0000 by Fisher's exact test).

After removing subjects that had baseline MN titers (and HAI titers) greater than 40, the geometric mean titers (GMT) were calculated in the remaining subjects on days 0 and 28 as shown in the following table. The GMT for the vaccine group rose to 247 (95 CI: 89-685) versus no rise in the placebo for a day 28 GMT of 9-6 (95 CI: 5-18). These calculations had no impact on the vaccine group, as none of the subjects had high initial MN or HAI titers. These results show that neutralizing antibody titers to influenza are generated by oral immunization, with a greater than 20-fold increase in the GMT after immunization in the vaccine-treated group.

| ASSAY | GROUP | N | GMT D0 | GMT D28 | GMFR | SC |
|---|---|---|---|---|---|---|
| HAI | Placebo | 11 | 8.3 | 8.8 | 1.1 | 0% |
| | Vaccine | 12 | 7.9 | 61.1 | 7.7 | 75% |
| MN | Placebo | 9 | 9.3 | 9.6 | 1.0 | N/A |
| | Vaccine | 12 | 8.6 | 247 | 29 | N/A |

In order to measure total antibody responses to HA, the numbers of circulating pre-plasma B cells in peripheral blood were measured by ASC assay on days 0 and 7 after immunization. Results show that ASCs could be reliably measured on day 7 in the vaccine-treated group (Figure 7D). Background ASCs were generally negligible on day 0. For the vaccine treated group, an average of 992 (+/- std err 209, 95% CI: 532-1452) IgG ASCs and 337 IgA ASCs (+/- std err 104, 95% CI: 117-580) each per 1 × 10⁶ PBMC were found for day 7, with only one subject out of 12 having no detectable ASC response. The placebo group had no IgA spots on day 7, but one subject had a high background smear and a measurable IgG ASC response with smaller spots than normally observed. The treated group was significantly different than placebo in terms of the ability to elicit an IgG or an IgA ASC response at day 7 (P=0·0007 and P=0.008 respectively by T Test).

Subjects were retrospectively measured for their anti-vector titers pre- and post-immunization. Following oral immunization, a few vaccine-treated subjects had an increase in neutralizing antibody responses to Ad5, which led to a 2·6-fold increase in the GM neutralizing antibody titers, compared to 1·0-fold GM fold rise in the placebo treated subjects. In the vaccine group, HAI and MN responses trended similarly for individual subjects. Eight subjects were Ad5 negative before immunization, and four were Ad5 positive before immunization. One subject that was Ad5 positive did not HAI seroconvert, however, one subject that was Ad5 positive had the highest increase in HAI titers (64 fold) of any of the subjects in the study. This same subject had a gain in MN titers of 362 fold without any increase in the Ad5 neutralizing antibody titers pre and post immunization. There was no observed correlation between starting Ad5 titers versus fold MN response (or HAI response) for the subjects immunized with the tablet vaccine.

Moreover, the presently disclosed tablet vaccine is stable at room temperature for greater than 270 days and can tolerate short-term excursions at higher temperatures, which makes this approach technically feasible.

### Example 5 Discussion

The US military conducted an independent study to measure the effects of their seasonal vaccine campaigns on neutralizing antibody responses in military personnel, and reported a MN Titer GMFR of 5.6 after trivalent inactivated vaccine (TIV) injection and a GMFR of 2.2 following live-attenuated influenza vaccine (LAIV) intranasal administration, after accounting for subjects that had MN titers above 40 to start (Faix et al. (2012) PloS one 7:e34581). In another study, the SC rate to H1N1 was found to be 45% for one injection of 45 ug of HA protein (without adjuvant) (Gordon et al. (2012) Vaccine 30:5407), while in another, the H1N1 vaccine was highly immunogenic with a 78% SC rate observed after 1 dose of a split vaccine (Greenberg *et al.* (2009) 361 :2405).

In contrast to the variable results observed with injected vaccines, in the present study, MN GMFR was calculated at 29 for the 12 vaccine treated subjects with 92% of subjects showing a greater than 4-fold rise in MN titers. In the present tablet study, the HAI SC rate among vaccine treated subjects was 75% with over 92% of subjects having a 4-fold rise in HAI titers (Figure 7A). MN titers were higher than the HAI titers. It is possible that the MN assay is more sensitive or that the oral rAd based vaccine elicits stronger neutralizing responses outside the head region than protein injected vaccines.

HAI responses are elicited with injected commercial vaccines, but HAI titers are known to wane. For example, non-HIV infected volunteers had a 67% drop in GMT HAI titers between 1 and 6 months post immunization (Crum-Cianflone et al. (2011) Vaccine 29:3183). Similarly, the percentage of seroprotected subjects dropped from 75% to 56% for HIV negative subjects that enrolled with seronegative HAI titers (≤1:10). Studies with pandemic influenza vaccines have also shown decreases in durability. In the AS03 avian influenza vaccine study, the GMT reached 563 after 2 vaccine doses, but at 6 months post immunization, the GMT had dropped to 18, a 96% decrease (Leroux-Roels et al. (2010) Vaccine 28:849). In the present tablet vaccine study, the percentage of seroprotected subjects remained constant at 75% at 1 and 6 months post immunization, and the HAI GMT titer drop was less dramatic showing only a 28% decrease (Figure 7B). One possibility is that the durability is better for vector-based vaccines because of enhanced T cell responses.

### Example 5 Materials and methods

*Clinical protocol and enrollment.* Subjects were pre-screened for Hemagglutination Inhibition (HAI) titers within 45 days of enrollment. In order to be eligible for study participation subjects had to have an initial HAI titer of ≤ 1:20, be between 18-49 years of age, and be in good health. The active phase of the trial was through day 28, with the follow-up phase for monitoring safety to continue for 1 year.

24 subjects were enrolled. All subjects that enrolled completed safety and immunogenicity assessments through the active phase, and through day 180 of the monitoring phase.

*Randomization and Masking.* The study was designed to evaluate the vaccine (VXA-A1·1) in 12 subjects at a single dose of 1 × 10¹¹ infectious units (IU) with 12 subjects given a placebo control. There were 3 sequentially enrolled sentinel vaccine-treated subjects, with each subject dosed no more frequently than one every 24 h. After a week of monitoring for vaccine-related toxicities, the remaining subjects in the treated cohort (9) were randomized along with 12 placebo controls. Randomization was performed by computer generated assignment, and study drug was distributed with concealed identity to the blinded staff by the unblinded pharmacist. All investigative site staff as well as persons directly involved with immunological assays or the assessment of clinical safety remained blind to treatment assignments. All subjects were blinded in the study.

*Vaccine.* The rAd vector (non-replicating Ad5) carries DNA which encodes the HA (A/CA/04/2009) transgene whose expression is driven by a CMV promoter and a molecular dsRNA hairpin driven by a separate promoter. GMP drug substance was produced in Wave bags (GE Healthcare, Waukesha, WI) at Lonza Biologicals (Houston, TX). Purification was performed by ion exchange chromatography, followed by buffer exchange. Purified vector was mixed with excipients, lyophilized, and then tableted at Lonza using microcrystalline cellulose and starch as tableting bulk. Tablets were enteric coated with Eudragit L100^{®} (Evonik Industries, Darmstadt, Germany) using a Vector Hi-Coater system (Vector Freund, Cedar Rapids, IA). The final product was released in one lot, and titered by standard IU assay at Lonza. Placebo was prepared as similarly sized and shaped tablets containing 150 mg of microcrystalline cellulose, without enteric coating.

*Endpoints.* The primary endpoint for this study is safety and the secondary endpoint is immunogenicity through the active phase, primarily by HAI titers and HAI seroconversions. Additional immunological endpoints include MN titers and ASCs. There were 5 adverse events in the placebo group and 4 in the vaccine group, all of which were grade 1 in severity. There were no serious adverse events reported in the study.

*PBMC isolation and cryopreservation.* Blood was collected in K₃ EDTA Vacutainer^{®} tubes (BD, Franklin Lakes, NJ) and PBMCs were isolated the same day using Lymphoprep^{™} tubes (Axis-Shield, Norway). PBMCs were frozen and thawed using serum free reagents according to the manufacturer's instructions (Cellular Technology Ltd [CTL], Shaker Heights, OH).

*Antibody Secreting cells (ASCs).* Enzyme linked immunosorbent (ELISpot) kits for IgG and IgA secreting B cells were performed according to manufacturer's instructions (Mabtech, Mariemont, OH). Cells were cultured (between 1·5 × 10⁴ to 5 × 10⁵ cells per well) in triplicate wells, in CTL-Test medium to optimize spots. HA protein (Protein Sciences Corp, Meriden, CT) was biotinylated and quantitated using a biotinylation kit (Pierce, Rockford, IL).

*Antibody assays.* HAI and Microneutralizing (MN) Titers were performed and were measured against MDCK derived A/CA/07/2009 and egg derived A/CA/07/2009 respectively. HAI and MN titers less than 10 were marked as 5 as suggested by regulatory advice.

*Statistical analysis.* Unpaired Students "t" tests were performed to test for significant differences between groups. A two-tailed Fisher's Exact test was used to determine if the observed frequencies were different for some analyses, as stated in the text. For both tests, *p* values of ≤0.05 were considered significant. 95 percent confidence intervals (95 CI) were provided for measured values.

It is understood that the examples and embodiments described herein are for illustrative purposes only and that various modifications or changes in light thereof will be suggested to persons skilled in the art and are to be included within the scope of the appended claims.

## Claims

1. An immunogenic composition for eliciting an immune response in a human comprising:
(i) an adenoviral vector, encoding an immunogenic polypeptide encompassed by (ii) an agent that directs delivery of the adenoviral vector to the ileum of the human, wherein agent (ii) is an enteric coating that has a threshold pH 5.8, 5.9, 6, or 6.1.

2. The immunogenic composition of claim 1, wherein the enteric coating has a threshold pH of 6.0.

3. The immunogenic composition of claim 1 or 2, wherein the enteric coating disintegrates at least 75% compared to its original thickness in 110 minutes at pH 5.8-6.8.

4. The immunogenic composition of any one of the foregoing claims, wherein the enteric coating comprises poly(methacrylic acid-co-methyl methacrylate) 1:1.

5. The immunogenic composition of any one of claims 1 to 3, wherein the enteric coating comprises Eudragit^{®} L-100.

6. The immunogenic composition of any one of claims 1 to 3, wherein the enteric coating comprises Eudragit^{®} L-100, triethyl citrate, and talc.

7. The immunogenic composition of claim 6, wherein the enteric coating comprises 1-4 parts Eudragit^{®} L-100, 1-2 parts triethyl citrate, and 1-2 parts talc.

8. The immunogenic composition of any one of the foregoing claims, wherein the adenoviral vector further encodes dsRNA.

9. The immunogenic composition of any one of the foregoing claims, wherein the composition is in the form of a compressed tablet.

10. The immunogenic composition of any one of the foregoing claims for use in a method for delivering the composition to the ileum of a human comprising orally administering the composition to the human.

11. The immunogenic composition of any one of claims 1-9 for use in a method of eliciting an immune response in a human comprising administering the composition to the human, wherein the immune response is specific for the immunogenic polypeptide.

12. The immunogenic composition for use of claim 11, wherein the administering results in production of neutralizing antibodies by the human.

## Patentansprüche

1. Immunogene Zusammensetzung zum Auslösen einer Immunantwort in einem Menschen, die umfasst:
(i) einen adenoviralen Vektor, der für ein immunogenes Polypeptid codiert, das von (ii) einem Mittel, das die Abgabe des adenoviralen Vektors an das Ileum des Menschen steuert, umschlossen wird, wobei das Mittel (ii) eine magensaftresistente Beschichtung ist, die einen Schwellenwert von pH 5,8, 5,9, 6 oder 6,1 aufweist.

2. Immunogene Zusammensetzung nach Anspruch 1, wobei die magensaftresistente Beschichtung einen Schwellen-pH-Wert von 6,0 aufweist.

3. Immunogene Zusammensetzung nach Anspruch 1 oder 2, wobei die magensaftresistente Beschichtung bei einem pH-Wert von 5,8 bis 6,8 innerhalb von 110 Minuten zu mindestens 75% im Vergleich zu ihrer ursprünglichen Dicke zerfällt.

4. Immunogene Zusammensetzung nach einem der vorhergehenden Ansprüche, wobei die magensaftresistente Beschichtung Poly(methacrylsäure-co-methylmethacrylat) 1:1 umfasst.

5. Immunogene Zusammensetzung nach einem der Ansprüche 1 bis 3, wobei die enterische Beschichtung Eudragit^{®} L-100 umfasst.

6. Immunogene Zusammensetzung nach einem der Ansprüche 1 bis 3, wobei die magensaftresistente Beschichtung Eudragit^{®} L-100, Triethylcitrat und Talkum umfasst.

7. Immunogene Zusammensetzung nach Anspruch 6, wobei die magensaftresistente Beschichtung 1-4 Teile Eudragit^{®} L-100, 1-2 Teile Triethylcitrat und 1-2 Teile Talkum umfasst.

8. Immunogene Zusammensetzung nach einem der vorhergehenden Ansprüche, wobei der adenovirale Vektor ferner für dsRNA codiert.

9. Immunogene Zusammensetzung nach einem der vorhergehenden Ansprüche, wobei die Zusammensetzung in Form einer gepressten Tablette vorliegt.

10. Immunogene Zusammensetzung nach einem der vorhergehenden Ansprüche zur Verwendung in einem Verfahren zur Abgabe der Zusammensetzung an das Ileum eines Menschen, das die orale Verabreichung der Zusammensetzung an den Menschen umfasst.

11. Immunogene Zusammensetzung nach einem der Ansprüche 1-9 zur Verwendung in einem Verfahren zur Auslösung einer Immunantwort bei einem Menschen, das die Verabreichung der Zusammensetzung an den Menschen umfasst, wobei die Immunantwort spezifisch für das immunogene Polypeptid ist.

12. Immunogene Zusammensetzung zur Verwendung nach Anspruch 11, wobei die Verabreichung zur Produktion von neutralisierenden Antikörpern durch den Menschen führt.

## Revendications

1. Composition immunogène pour provoquer une réponse immunitaire chez un humain comprenant :
(i) un vecteur adénoviral, codant pour un polypeptide immunogène englobé par (ii) un agent qui dirige l'administration du vecteur adénoviral vers l'iléon de l'humain, l'agent (ii) étant un enrobage entérique qui a un pH seuil de 5,8, 5,9, 6, ou 6.1.

2. Composition immunogène selon la revendication 1, dans laquelle l'enrobage entérique a un pH seuil de 6,0.

3. Composition immunogène selon la revendication 1 ou 2, dans laquelle l'enrobage entérique se désintègre à au moins 75 % par rapport à son épaisseur initiale en 110 minutes à pH 5,8 à 6,8.

4. Composition immunogène selon l'une quelconque des revendications précédentes, dans laquelle l'enrobage entérique comprend du poly(acide méthacrylique-co-méthacrylate de méthyle) 1 : 1.

5. Composition immunogène selon l'une quelconque des revendications 1 à 3, dans laquelle l'enrobage entérique comprend de l'Eudragit ^{®} L-100.

6. Composition immunogène selon l'une quelconque des revendications 1 à 3, dans laquelle l'enrobage entérique comprend de l'Eudragit ^{®} L-100, du citrate de triéthyle et du talc.

7. Composition immunogène selon la revendication 6, dans laquelle l'enrobage entérique comprend 1 à 4 parties d'Eudragit ^{®} L-100, 1 à 2 parties de citrate de triéthyle et 1 à 2 parties de talc.

8. Composition immunogène selon l'une quelconque des revendications précédentes, dans laquelle le vecteur adénoviral code en outre pour l'ARNdb.

9. Composition immunogène selon l'une quelconque des revendications précédentes, dans laquelle la composition se présente sous la forme d'un comprimé compressé.

10. Composition immunogène selon l'une quelconque des revendications précédentes destinée à être utilisée dans un procédé pour délivrer la composition à l'iléon d'un humain comprenant l'administration orale de la composition à l'humain.

11. Composition immunogène selon l'une quelconque des revendications 1 à 9, destinée à être utilisée dans un procédé pour provoquer une réponse immunitaire chez un humain comprenant l'administration de la composition à l'humain, dans laquelle la réponse immunitaire est spécifique du polypeptide immunogène.

12. Composition immunogène à utiliser selon la revendication 11, dans laquelle l'administration entraîne la production d'anticorps neutralisants par l'humain.
